# EUROPEAN PATENT SPECIFICATION

(11) **EP 3 485 103 B1**
(45) Date of publication and mention of the grant of the patent: **07.09.2022**
(21) Application number: 17831614.7
(22) Date of filing: 16.07.2017
(51) Int. Cl.: B65D 83/00, B65D 83/14, B65D 83/16, B65D 83/26, B65D 83/38, A61M 11/08, A61M 35/00, E03D 9/08, A47K 5/12, A61M 15/00, B05B 15/70

(54) **A WASH, CLEAN, AND DRY SYSTEM WITH REMOVABLE SPRAY CANISTER DEVICE**
WASCH-, REINIGUNGS- UND TROCKNUNGSSYSTEM MIT ABNEHMBARER SPRÜHKANISTERVORRICHTUNG
SYSTÈME DE LAVAGE, DE NETTOYAGE ET DE SÉCHAGE DOTÉ D'UN DISPOSITIF DE BOMBE DE PULVÉRISATION AMOVIBLE

(30) Priority: 16.07.2016 US 201662363232 P; 13.07.2017 US 201715649564
(43) Date of publication of application: 22.05.2019
(73) Proprietor: Bemis Manufacturing Company, Sheboygan Falls, WI 53085 (US)
(72) Inventor: SCHWAB, Brian, East Mountain, NY 12060 (US); Peng, Shao-Yu, Huatan Township Changhua County (TW); Murray, Brian, Albany, NY 12203 (US)
(74) Representative: King, Alex
(86) International application number: PCT/US2017/042288
(87) International publication number: WO 2018/017437

(56) References cited:
- JP-A- 2003 342 993
- JP-A- 2007 321 443
- US-A- 5 335 855
- US-A- 5 504 948
- US-A1- 2013 133 131
- US-A1- 2013 133 131
- US-A1- 2015 000 025
- US-B1- 6 167 577
- US-B1- 6 339 852
- US-B1- 6 973 679
- US-B1- 8 776 278

## Description

### BACKGROUND OF THE INVENTION

The present invention generally relates to a spray device and particularly to a spray canister device for the delivery of water, medication, solutions and/or a pharmaceutical formulation to a surface area of a human subject using a bidet or other modern toilet seat system. More specifically, aspects of the present invention provide a method and device for delivering and applying water, medication, and/or a cleaning solution to a region of a human body (e.g., genital or anal area, intimate parts, perianal region).

### Description of the Related Art

People who have problems controlling their urine or bowels and suffer from incontinence are at risk of skin problems around the buttocks, hips, genitals, and the area between the pelvis and rectum (perineum). Excess moisture in these areas may result in skin problems (e.g., redness, peeling, irritation, and yeast infections, etc.), and if the person spends most of his or her day in a wheelchair, or bed, it is likely that bedsores may also develop. Such skin problem may be worse if the person uses diapers and other products, which allow urine or stool to be in constant contact with the skin. As such, special care by cleaning and drying the area right away after urinating or having a bowel movement, and/or cleaning the skin with mild, dilute soap and water then rinsing well and gently patting dry. In addition, moisturizing creams can help keep the skin moist. Also, a skin sealant or moisture barrier, barrier creams or ointments that contain occlusive, barrier-type topical, such as zinc oxide, lanolin, or petrolatum can form a protective barrier on the skin. Some skin care products, often in the form of a spray or a towelette, create a clear, protective film over the skin. A doctor or nurse can recommend barrier creams to help protect the skin.

There are a wide variety of ointments, creams or lotions known and available in the market for the treatment of diaper rash or incontinence. Most of these products include ingredients that offer some beneficial property to the product, for example, by acting as a water repellant, emollient, neutralizer or antibiotic. Examples of ingredients that are commonly included in such ointments are mineral oil, silicone fluids (e.g., dimethicone and cyclomethicone), petrolatum, cod liver oil, lanolin, zinc oxide, talc, calamine, kaolin, topical starch and allantoin. For example, Desitin^{®} ointment (*Pfizer, Inc.)* is probably the most common topical used in treating diaper rash. It contains common barrier materials (zinc oxide and petrolatum) and additionally contains two common skin conditioning agents (cod liver oil and lanolin).

In applying skin protection paste, ointments, barrier sprays, lotions, solutions, and fluids to an individual's skin. If the individual is alone, this is impossible since an individual cannot adequately apply skin protection fluid onto hard-to-reach areas of his or her own back and bottom. Accordingly, it is necessary to utilize the services of a second individual to apply the skin protection fluid on hard-to-reach areas such as the back and upper neck. Thus, there is a need for using a spray device, which applies a barrier materials, medicated solutions, fluids, protectants, suspensions, or paste to the skin area of a person, to be incorporated into a bidet seat cleaning systems have water spray nozzle for washing and cleaning private parts of the person.

### SUMMARY OF THE INVENTION

The present invention generally includes a method and a system for washing, cleaning and drying genital and anal area of a human body. The system includes a toilet seat assembly with a bidet assembly having a spray canister device for spraying the surface region with a solution, such as a skin protecting barrier solution, a cleaning solution or a medicated solution. The spray canister device is removable in and out of the system, very easy to carry, easy to be connected to auxiliary parts, and easy to install and use.

In addition, the bidet assembly further includes a spray nozzle assembly and a drying nozzle assembly, which are adapted to wash, clean and dry the region of the human body in three dimensional moments. The removable spray canister device with the removable sleeved cover element is thus easy to carry and be re-filled with new solutions.

One embodiment of the invention provides an apparatus for storing a first solution and spraying the first solution onto a region of the human body. The apparatus includes a toile seat assembly having a seat cover and a bidet seat, wherein the bidet seat comprises a seat body, a seat base, a bidet housing for housing a bidet assembly therein, a bidet spray cover, and a bidet housing cover. In one aspect, the bidet housing cover is positioned at a surface of the bidet housing. In another aspect, the bidet spray cover is capable of being in an open position and a closed position and adapted to cover the bidet assembly housed within the bidet housing. In a further aspect, the spray canister device can be movably insert into and out of a toilet seat assembly of the system and is easy to operate and use.

In still another aspect, the bidet assembly includes a removable spray canister device adapted to store the first solution and be inserted in and out of the base housing cover, wherein the removable spray canister device includes a sleeved cover element and a canister element. In addition, the first solution is stored within the canister element of the removable spray canister device and the first solution is sprayed out by triggering the trigger bar of the sleeved cover element against the canister element and moving the bottom portion of the canister element to be closer to the opening of the sleeved cover element. Further, the sleeved cover element includes a handle grip adapted to handle the removable spray canister device in and out of the base housing cover.

In one embodiment, the bidet assembly includes the removable spray canister device, a spray nozzle assembly, and a drying nozzle assembly. In one aspect, the spray nozzle assembly includes a spray nozzle housing adapted to house one or more spray nozzle units with one ore more spray nozzle wands that are retractable and adapted to deliver a second solution to the region of the human body, and a first driving motor being connected to the one or more spray nozzle wands and adapted for moving the one ore more spray nozzle wands in retracting-and-extending motion. Further, the spray nozzle assembly may also include one or more steering mechanisms, and a second driving motor being connected to the one or more spray nozzle wands and adapted for moving the one or more spray nozzle wands in three-dimensional circular rotational motion.

In another aspect, the dying nozzle assembly includes one or more drying nozzle units with one ore more retractable air blower wands adapted to deliver air at a predetermined temperature to the region of the human body, and a fan connected to the one or more drying nozzle units. Further, the dying nozzle assembly may also include a first driving motor being connected to the one or more drying nozzle units and adapted for moving the one ore more retractable air blower wands in retracting-and-extending motion, and a second driving motor being connected to the one or more drying nozzle unit and adapted for moving the one or more retractable air blower wands in three-dimensional circular rotational motion.

In another embodiment, the toilet seat assembly of the invention may also include a medicine delivery assembly having one or more medicine storage cartridges, wherein the medicine delivery assembly is adapted to deliver one or more medicine-containing solutions stored at the one or more medicine storage cartridges. In one aspect, the medicine delivery assembly is coupled to a spray nozzle channel of a spray nozzle assembly.

In another embodiment, the apparatus of the invention further includes one or more control units adapted to receive an user input and, based on the user input, to direct movements of the bidet assembly to be retractable out of the bidet housing when the bidet cover is in a open position and retractable inside the bidet housing when the bidet cover is in a closed position, wherein the one or more control units is selected from the group consisting of a remote control unit, a touch screen control unit, a joystick type control unit, a hand-held control unit, a steering-wheel type control unit, a built-in control unit adjacent the toilet seat assembly, a front end touch control unit, and combinations thereof. In one aspect, a front end touch control unit positioned at a front end of the seat body is used to control the movements of the bidet assembly, and adapted to control opening and closing movements of the bidet cover. In another aspect, the front end touch control unit is a single button with electric circuits therein to be adapted to control the movements of the bidet assembly and the opening and closing movements of the bidet cover, wherein the user input comprises repeated touching of the single button.

A method of using an apparatus to deliver one or more solutions to a region of a human body is also provided. The method includes washing an area of the region with water using the apparatus having a toile seat assembly for housing a bidet assembly therein, wherein the bidet assembly includes a removable spray canister device, a spray nozzle assembly, and a drying nozzle assembly. In one aspect, the method also includes controlling the movements of the bidet assembly and the opening and closing movements of a bidet cover using one or more control units, drying the area of the region using an drying nozzle assembly of the bidet assembly, and applying a spray of a first solution onto the area of the region using the removable spray canister device which stores the first solution therein.

The method further includes drying the area of the region after applying the spray of the first solution onto the area of the region using the removable spray canister device. In still another aspect, the method further includes repeatedly touching of the single button of the front end touch control.

### BRIEF DESCRIPTION OF THE DRAWINGS

So that the manner in which the above recited features of the present invention can be understood in detail, a more particular description of the invention, briefly summarized above, may be had by reference to embodiments, some of which are illustrated in the appended drawings. It is to be noted, however, that the appended drawings illustrate only typical embodiments of this invention and are therefore not to be considered limiting of its scope, for the invention may admit to other equally effective embodiments.
Figure 1 is a perspective view of a wash, clean and dry system with a spray canister device stored within a bidet housing cover of a bidet assembly of a toilet seat assembly, the spray canister device being adapted to be movable in and out via a bidet housing cover (in a closed position) according to one embodiment of the invention.
Figure 2A is a front side view of the wash, clean and dry system of Figure 1, where a portion of the spray canister device and a portion of the bidet assembly are extended out of a spray cover of the toilet seat assembly according to another embodiment of the invention.
Figure 2B is an inside view of the bidet assembly of Figure 2A, showing the spray canister device, a spray nozzle assembly, and a drying nozzle assembly according to another embodiment of the invention.
Figure 3 is a top view of the toilet seat assembly of Figure 1, showing a front end control unit and the bidet housing cover positioned in an open position for receiving the spray canister device into the interior (partially shown) of a bidet housing of the toilet seat assembly of the wash, clean and dry system according to one embodiment of the invention.
Figure 4A is an outside view of an exemplary spray canister device with a removable sleeved cover element and a canister element according to another embodiment of the invention.
Figure 4B is a top view of an exemplary spray canister device being movable inserted into a bidet assembly of a wash, clean and dry system according to still another embodiment of the invention.
Figure 4C is a side view of an exemplary spray canister device being inserted into a bidet assembly of a wash, clean and dry system in a direction "R" according to still another embodiment of the invention.
Figure 4D is a side view of an exemplary spray canister device after being inserted into a bidet assembly of a wash, clean and dry system according to still another embodiment of the invention.
Figure 4E is a top view of an exemplary spray canister device after being inserted into a bidet assembly of a wash, clean and dry system, where the spray canister device, a spray nozzle wand, and an blower wand are all in an extended position according to still another embodiment of the invention.
Figure 5A is a top view of an exemplary spray canister device after being inserted into a bidet assembly of a wash, clean and dry system, where the spray canister device is in an extended position, but a spray nozzle wand, and an blower wand are both retracted according to still another embodiment of the invention.
Figure 5B is a top view of an exemplary spray canister device right after being inserted into a bidet assembly of a wash, clean and dry system, yet the spray canister device is still in a retracted position according to still another embodiment of the invention.
Figure 5C is a partial cross-sectional view of an exemplary spray canister device after being inserted into a bidet assembly of a wash, clean and dry system, where the spray canister device is in an extended position according to still another embodiment of the invention.
Figure 6A is a perspective view of an exemplary bidet assembly, where a spray canister device is movably inserted into a bidet assembly but the spray canister device and a drying nozzle assembly are both retracted, and only a spray nozzle wand is in an extended position according to yet another embodiment of the invention.
Figure 6B is a perspective view of a spray nozzle assembly, showing a spray nozzle wand in a retracted position within a spray nozzle housing according to yet another embodiment of the invention.
Figure 6C is a partial cross-sectional view of a spray nozzle assembly, showing a spray nozzle wand is in an extended position according to yet another embodiment of the invention.
Figure 7A is a perspective view of an exemplary bidet assembly, where a spray canister device is movably inserted into a bidet assembly but a spray nozzle assembly and the spray canister device are both retracted, and only an air blower wand of a drying nozzle assembly is in an extended position according to yet another embodiment of the invention.
Figure 7B is a perspective view of a drying nozzle assembly, showing an air blower wand in a retracted position within an air blower housing according to yet another embodiment of the invention.
Figure 7C is a partial cross-sectional view of a drying nozzle assembly, showing an air blower wand is in an extended position according to yet another embodiment of the invention.
Figure 8 illustrates a method of using a wash, clean and dry system to deliver one or more solutions to a region of a human body according to one or more embodiments of the invention.

### DETAILED DESCRIPTION

The present invention includes an apparatus for providing easy washing, drying, cleaning, and applying a protective solution/cream onto a person's bottom while using a toilet. The apparatus can be placed directly over a rim of a toilet bowl or a seat of a toilet seat system. A method and a wash, clean and dry system are provided for washing, cleaning, drying and protecting a surface region of a human body is also provided. The apparatus includes a toilet seat assembly with a bidet assembly having a spray canister device for spraying the surface region with a solution, such as a skin protecting barrier solution, a cleaning solution or a medicated solution. In one aspect, the spray canister device can be movably insert into and out of the toilet seat assembly and is easy to operate and use. In addition, the bidet assembly further includes a spray nozzle assembly and a drying nozzle assembly, which are adapted to wash, clean and dry the region of the human body in three dimensional moments. The removable spray canister device with the removable sleeved cover element is thus easy to carry and can be re-filled with new solutions or protective creams.

Embodiments of the present invention generally relate to a spray device and particularly to a spray canister device for the delivery of a skin protecting barrier solution in metered doses to a surface area of a human subject. More specifically, aspects of the present invention provide a method and device for delivering and applying water, medication, a cleaning solution (e.g., a bath cleaning solution, an enzymatic solution, a disinfectant solution, *etc.),* and/or skin protecting barrier solution to a region of a human body (e.g., genital or anal area, intimate parts, perianal region).

Exemplary skin protecting solution include, but are not limited to, skin protectants, ointments, mineral oil, silicone fluids, dimethicone, cyclomethicone, petrolatum, cod liver oil, lanolin, zinc oxide, talc, calamine, kaolin, topical starch and allantoin, barrier materials, zinc oxide, skin moisturizers, skin lotions, moisturizing creams, skin sealants, water, medication, cleaning solutions, moisture barriers, medicaments, a pharmaceutical formulation, and combinations thereof.

In one embodiment, the spray canister device can be a manually operated, hand-held, rechargeable, liquid spray device having a non-pressurized, refillable or non-refillable container/canister element. In an alternative embodiment, the spray canister device can be inserted into a bidet assembly of a toilet seat assembly.

The spray canister device can a charging mechanism whereby, upon shaking or moving back and froth of one part relative to another part (*e.g.*, shaking or moving or pressing back and froth of a sleeved cover element against a canister element of the spray canister device, a barrier solution or ointment stored within the canister element is sprayed out of an opening at a top portion of the spray canister device.

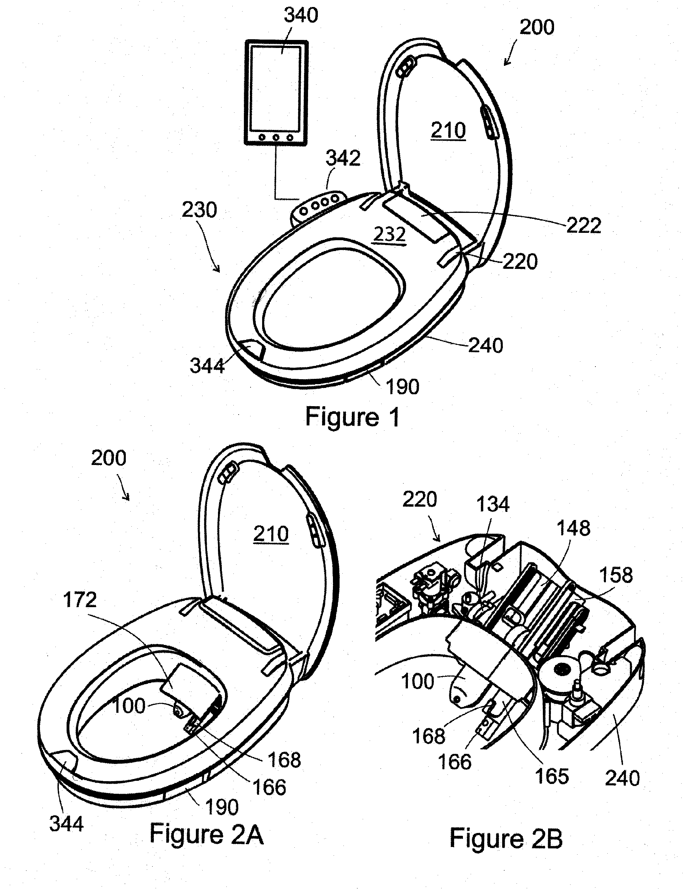

Figure 1 is a perspective view of a wash, clean and dry system having a toilet seat assembly 200 and a bidet assembly 150 with a spray canister device 100 adapted to store a first solution of a skin protectant or barrier spray ointment and spray the first solution onto a region of the human body. The toilet seat assembly 200 includes a seat cover 210 and a bidet seat 230. The bidet seat 230 includes a seat body 232, a seat base 240, a bidet housing 220 for housing the bidet assembly 150 therein, and a bidet housing cover 222.

In one embodiment, the bidet housing cover 222 is positioned at a surface of the bidet housing 220. In one aspect, the spray canister device 100 can be installed into the bidet assembly 150 by inserting the spray canister device 100 movably in and out of the base housing 220 via the base housing cover 222. In Figure 1, the spray canister device 100 is stored within the bidet housing 220 of the bidet seat 230 when the bidet housing cover 222 is in a closed position

As shown in Figure 1, the wash, clean and dry system may further include one or more control units adapted to receive an user input and, based on the user input, to direct movements of the bidet assembly 150. Suitable control units may include, but are not limited to, remote control units, touch screen control units, joystick type control units, hand-held control units, steering-wheel type control units, built-in control units adjacent the toilet seat assembly, front end touch control units, and combinations thereof. For example, in Figure 1, a control unit 340, a control unit 342, and a front end touch control unit 344, can be used alone or in combination to control the various movements and functions of the toilet seat assembly 200 of the wash, clean and dry system.

In one aspect, the bidet assembly 150 is normally stored within the bidet housing 220 in a resting, retracted position and is covered by the bidet spray cover 172. In one embodiment, the bidet assembly 150 generally includes the spray canister device 100, a spray nozzle assembly 160, and a drying nozzle assembly 170.

Figure 2A is a front side view of the toilet seat assembly 200, where a portion of the spray canister device 100 and a portion of the bidet assembly 150 are extended out of a bidet spray cover 172 of the toilet seat assembly 200. Figure 2B is an inside view of the bidet assembly 150 of the toilet seat assembly 200 of Figure 2A. In one embodiment, the spray canister device 100 within the bidet assembly 150 is adapted to move back and forth along a delivery track 148. In anther embodiment, the spray nozzle assembly 160 and the drying nozzle assembly 170 are adapted to extend and retract along a delivery track 158.

In another aspect, the bidet assembly 150 is adapted to be retractable out of the bidet housing 220 when the bidet spray cover 172 is in a open position, as shown in Figure 2A, and retractable inside the bidet housing when the bidet spray cover 172 is in a closed position (See, Figure 1). In one embodiment, the control units 340, 342 and/or the front end touch control unit 344 positioned at a front end of the seat body are adapted to control the movements of the bidet assembly 150, and adapted to control opening and closing movements of the bidet spray cover 172.

In another embodiment, the front end touch control unit 344 is a single button with electric circuits therein to be adapted to control the movements of the bidet assembly 150 and the opening and closing movements of the bidet spray cover 172. In still another embodiment, an user input may include repeated touching of the single button of the front end touch control unit 344 to progress a series of movement control commands to control the bidet assembly 150.

In operation, a human user can easily to manually install the spray canister device 100 into the toilet seat assembly 200 by hand using the handle grip 112 to slip the spray canister device 100 through the opening of the bidet housing cover 222 and glide the spray canister device 100 into the delivery track 158 to secured the spray canister device 100 in a secured retracted position within the bidet assembly 150.

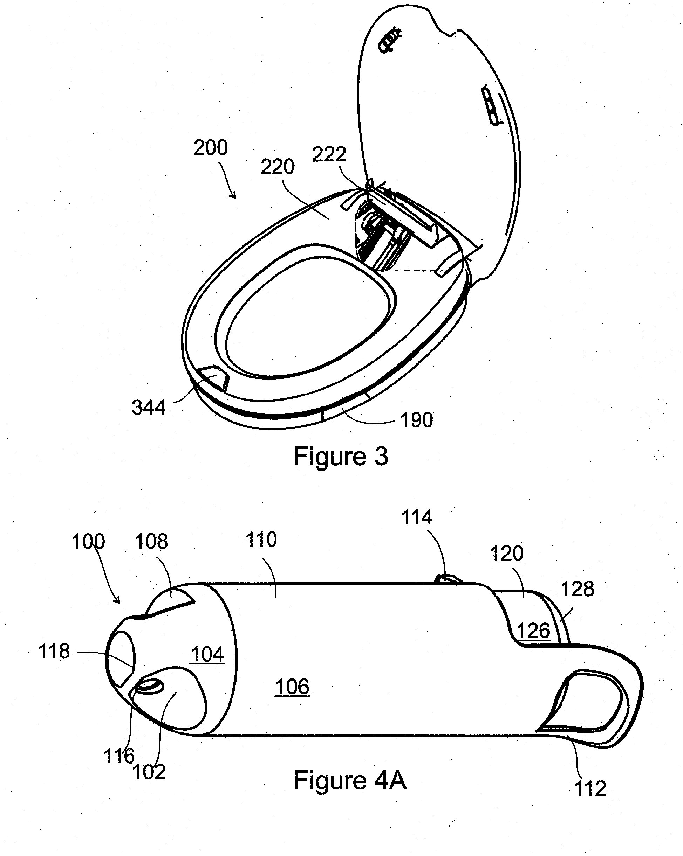

Figure 3 is a top view of the toilet seat assembly 200 of Figure 1, showing the front end control unit 344 and the bidet housing cover 222 positioned in an open position for receiving the spray canister device 100 into the interior (partially shown) of the bidet housing 220 of the toilet seat assembly 200. As shown in Figure 1 and Figure 3, the bidet housing cover 222 is adapted to cover the spray canister device 100 housed within the bidet housing 220 and capable of being in a closed position and an open position, respectively.

Figure 4A illustrates the spray canister device 100, which includes a sleeved cover element 110 and a canister element 120. The sleeved cover element 110 may include a top cap portion 104 and a sleeve portion 106. The top cap portion 104 may include a top body portion 108 and a cut-out portion 102. The top cap portion 104 may include a ring portion forming the base of the top cap portion 104 so that the shape of the ring portion conforms to the shape of the sleeve portion 106. In one aspect, the cut-out portion 102 includes one or more openings 116 for spraying out droplets or mist of a skin protecting cream, a cleaning solution or a medicated solution or ointment stored inside the canister element 120.

For example, the canister element 120 can be used to store a cleaning solution or a medicated solution to a surface area of a human body to be sprayed out by an opening 116 of the sleeved cover element 110 of the spray canister device 100 so that it is easy to operate and use. Examples of ingredients that are commonly included inside the inner portion (e.g., the canister element 120) of the spray canister device 100 are ointment, paste or solutions of mineral oil, silicone fluids (e.g., dimethicone and cyclomethicone), petrolatum, cod liver oil, lanolin, zinc oxide, talc, calamine, kaolin, topical starch and allantoin. For example, Desitin^{®} ointment (*Pfizer, Inc.)* is probably the most common topical used in treating diaper rash and other rash. It contains common barrier materials (zinc oxide and petrolatum) and additionally contains two common skin conditioning agents (cod liver oil and lanolin).

As shown in Figure 4A, the sleeved cover element 110 may include a handle grip 112, and a trigger bar 114. In addition, the canister element 120 may include a canister body 126 and a bottom portion 128. The handle grip 112 of the sleeved cover element 110 is used for easy-to-grip, easy to carry, and easy to hang the whole device, among others, and the trigger bar 114 of the sleeved cover element 110 is used to assist in coupling to one or more drive mechanisms and/or steering mechanism of the bidet assembly 150 for shaking (e.g., moving back and forth within the bidet assembly 150) so as to atomize and spray the content inside the canister body 120 out of the opening 116. In addition, the handle grip 112 of the sleeved cover element 110 is adapted to handle the spray canister device 110 in and out of the bidet housing cover 222. As such spray canister device 100 as described herein is easy-to-handle and easy to apply and spray fluids, solutions, suspensions, or paste of a barrier chemical or medicament to the skin area of a person.

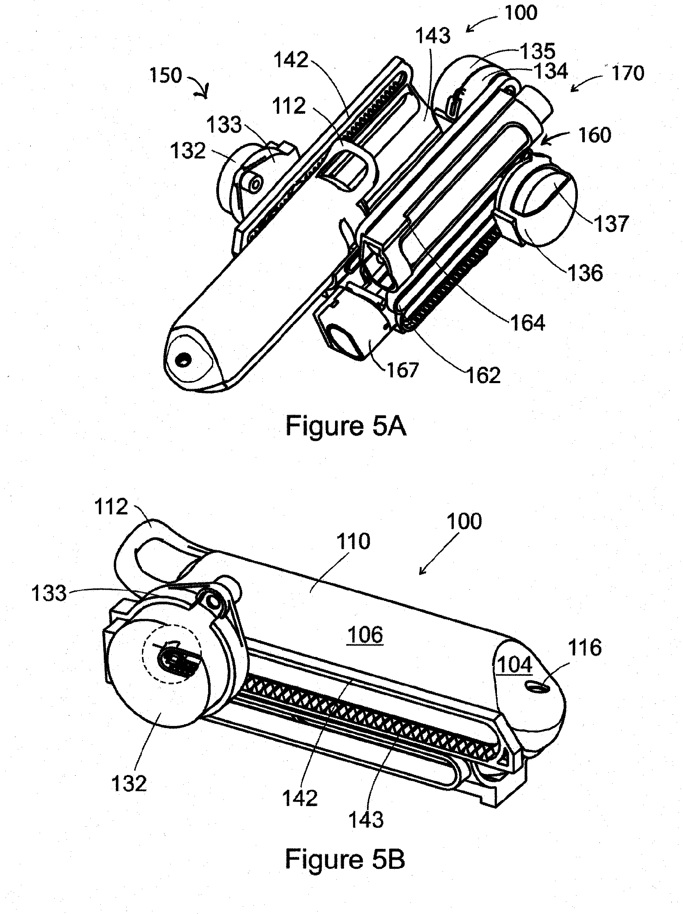

In one embodiment, the first solution is stored within the canister element 120 of the spray canister device 100 and the first solution is sprayed out by triggering the trigger bar 114 of the sleeved cover element 110 onto one ore more drive mechanisms (e.g., trigger drive mechanism 142, as shown in Figure 5B) positioned along a delivery track 143 of the bidet assembly 150 and moving back and froth and pushing the bottom portion 128 of the canister element 120 to be closer to the opening 116 of the sleeved cover element 110.

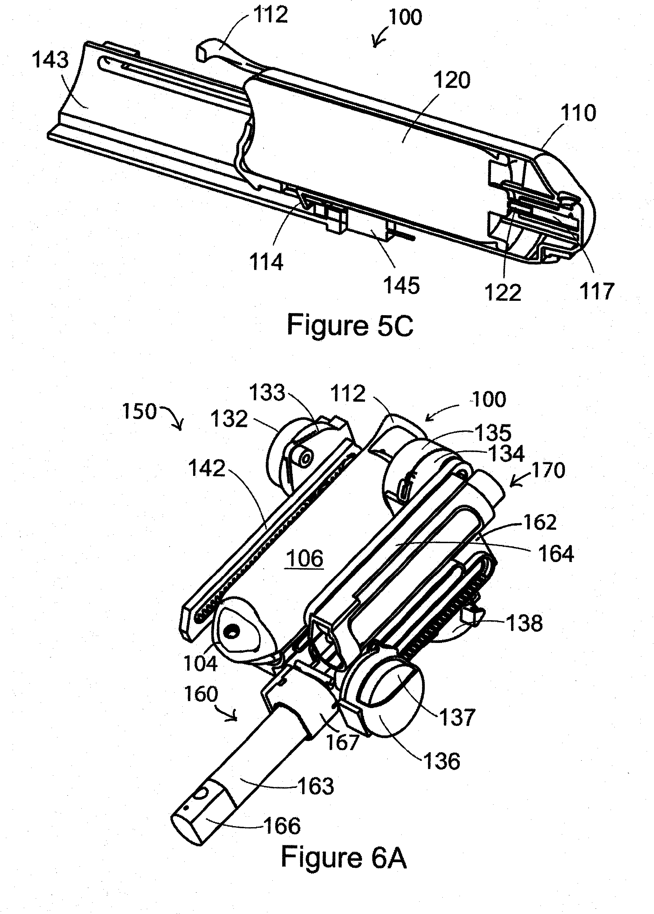

The sleeved cover element 110 of the spray canister device 100 may further include the top body portion 108 with channels 117 (as shown in Figure 5C) therein and a top flat surface portion 118 for easy handling. In one aspect, the interior content of the canister element 120 is sprayed out by pushing down the top flat surface portion 118 of the sleeved cover element 110 to atomize the content of the barrier spray, cleaning or medicated solution inside the canister element 120 and spray out droplets or mists of the barrier materials, medicaments or cleaning solutions.

In one embodiment, the canister element 120 may include a top outlet, a ring portion, the canister body 126, and the bottom portion 128. In one aspect, the canister body 126 of the canister element 120 is covered by the sleeved portion 106 of the sleeved cover element 110 and the top outlet 122 is fitted to channels 117 inside the top cap portion 104 of the sleeved cover element 110.

Conveniently, the spray canister device can be easily installed into the bidet assembly 150 by hand using the handle grip 112 to slip the spray canister device 100 through the opening of the bidet housing cover 222 and glide the spray canister device 100 into the delivery track 148 within the bidet assembly 150 to secured the spray canister device 100 in a secured retracted position via coupling the trigger bar 114 to the trigger drive mechanism 142 of the bidet assembly 150.

In one aspect, the spray canister device 100 is operated by moving the trigger bar 114 of the sleeved cover element 110 against the canister element 120 (e.g., move in relatively opposite directions) and moving the bottom portion 128 of the canister element in a direction toward the top cap portion 104 so as to be closer in distance to the opening 116 of the sleeved cover element 110. After a number of back and forth movements and/or shaking movement, the interior content of the canister element 120 is mixed/shaken and then atomized and sprayed out of the spray canister device 100 via the opening 116.

One example of the spray canister device 100 is described in detail in co-pending U.S. patent application serial number 15/649,564, filed July 13, 2017,titled "Spray Canister Device with Removable Sleeved Cover". The disclosure of this US patent application is hereby incorporated by reference.

In another aspect, the interior content of the canister element 120 is sprayed out by inserting the spray canister device 100 into the bidet assembly 150 via the bidet housing cover 222 and shaking the content of the canister element 120 by moving the spray canister device 100 back and froth inside the bidet assembly 150 to atomize the content of the ointment solution inside the canister element 120 and spray out droplets or mists of the barrier materials, medicaments or cleaning solutions.

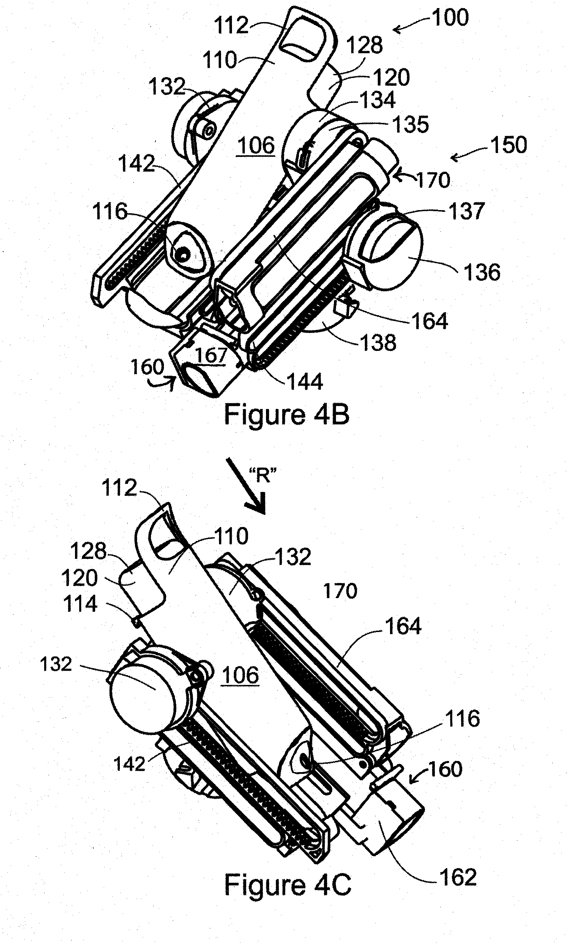

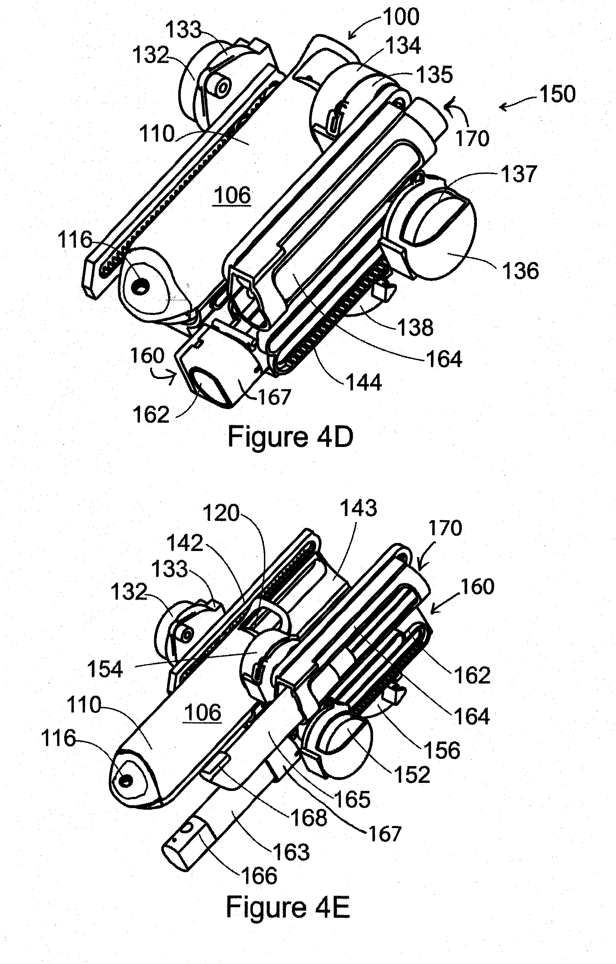

Figure 4B shows the spray canister device 100 being inserted into the bidet assembly 150, and Figure 4C shows the spray canister device 100 is capable of moving within the bidet assembly in a direction "R" according. Figure 4D shows the spray canister device 100 after being inserted into the bidet assembly 150 in a resting retracted position, and Figure 4E shows the spray canister device 100 after being inserted into the bidet assembly 150 and the spray canister device 100, a spray nozzle wand 163, and an blower wand 165 are all in an extended position.

Figure 5A shows the spray canister device 100 after being inserted into the bidet assembly 150, where the spray canister device 100 is in an extended position, but the spray nozzle wand 163 and the blower wand 165 are both retracted. Figure 5B shows only the spray canister device 100 after being inserted into the bidet assembly 150, yet the spray canister device 150 is still in a retracted position. Figure 5C is a partial cross-sectional view of only the spray canister device 100 after being inserted into the bidet assembly 150, where the spray canister device 100 is in an extended position.

As shown in Figure 5C, one or more channels 117 inside the top cap portion of the sleeved cover element 110 are used to deliver a cleaning or medicated solution inside the canister element 120 and spray droplets or mists of the barrier materials, medicaments or cleaning solutions out of the opening 116.

As shown in Figures 4A-4E and Figures 5A-5C, the spray canister device 100 within the bidet assembly 150 is able to move back and forth in the direction "R" between the retracted position and the extended position by securing the trigger bar 114 onto a trigger drive mechanism 142 with the assistance of a solenoid 145. Accordingly, the spray canister device 100 is adapted to move back and forth along a delivery track 143 with the assistance of a motor 133 and a motor drive mechanism 132.

In one embodiment, the spray nozzle assembly 160 of the bidet assembly 150 includes a spray nozzle housing 162 with one or more spray nozzle wands 163 positioned therein for delivering one or more water or various kinds of washing and cleaning solutions. Alternatively, the spray nozzle housing 162 may house one spray nozzle wand 163 with one or more spray channels therein, each spray channel being provided for flowing a separate channel of water, washing and/or cleaning solutions therein. The predetermined temperature of water or any washing solution within the spray nozzle wands 163 can be adjusted to be set at between about 20 °C to about 60 °C.

Figure 6A is a perspective view of an exemplary bidet assembly, where the spray canister device 100 is movably inserted into the bidet assembly 150 and only the spray nozzle wand 163 is in an extended position with a spray nozzle head 166 with one or more spray outlets facing upward. However, the spray canister device 100 and the drying nozzle assembly 170 are both retracted.

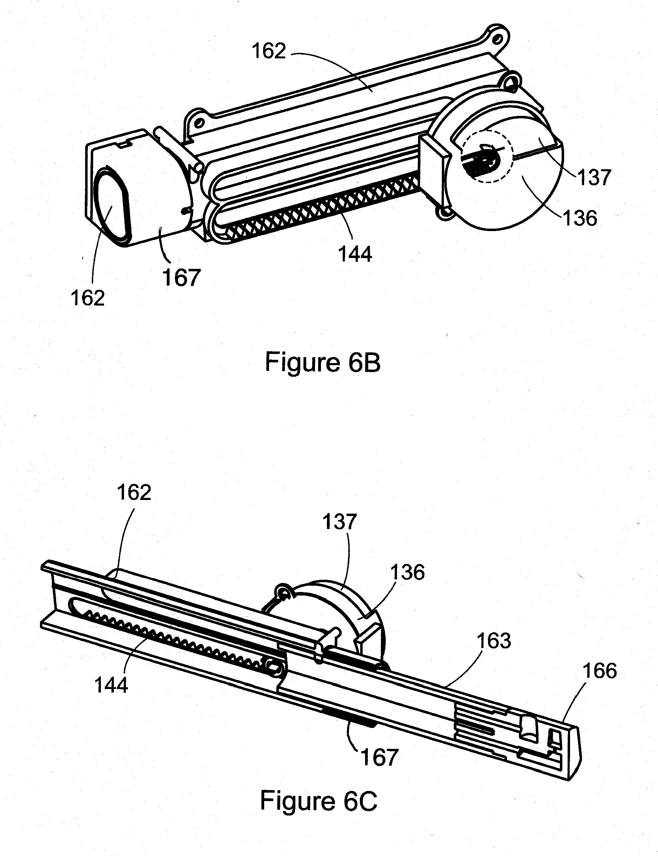

Figure 6B is a perspective view of another example of the spray nozzle assembly 160, showing the spray nozzle wand 163 in a retracted position within a spray nozzle housing 162. Figure 6C is a partial cross-sectional view of yet another example of the spray nozzle assembly 160, showing the spray nozzle wand 163 and the spray nozzle head 166 are in an extended position with one ore more channels therein.

In another embodiment, the drying nozzle assembly 170 of the bidet assembly 150 includes an air blower housing 164 to house one or more drying nozzle units, each drying nozzle unit is adapted to include one ore more air blower wands 165 positioned to be retractable to deliver air at a predetermined temperature to the region of the human body. Each of the air blower wands 165 is adapted to be connect to a fan for delivering air therein. The predetermined temperature of the air within the one or more air blower wands 165 can be adjusted to be set at between about 20 °C to about 60 °C.

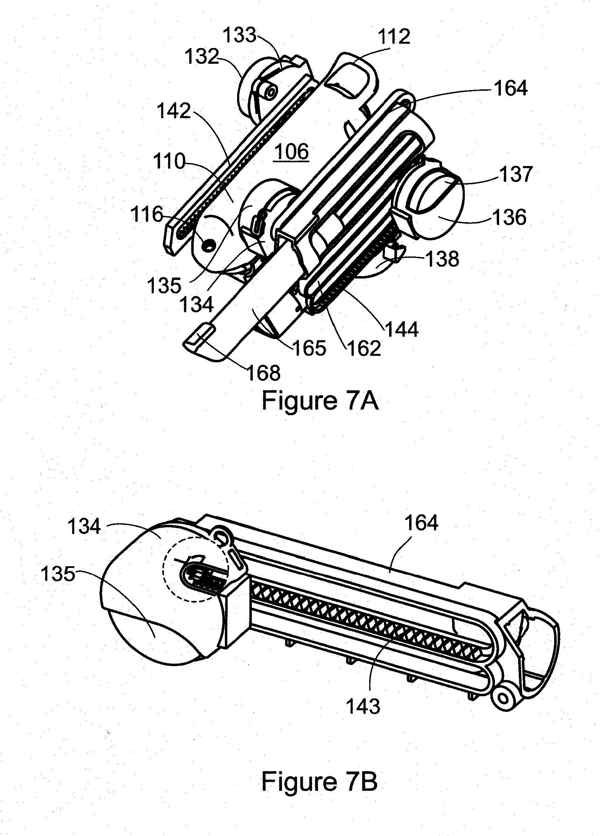

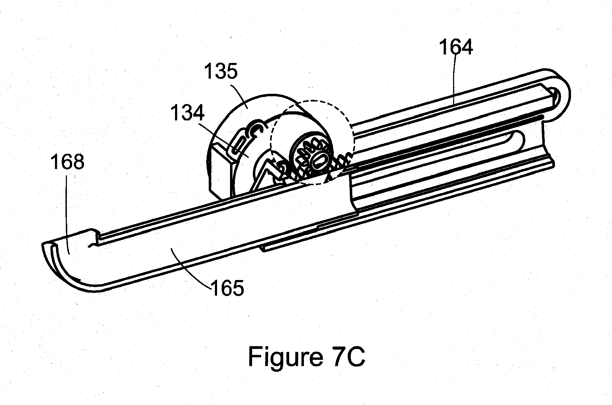

Figure 7A is a perspective view of one example of the bidet assembly 170, where the spray canister device 100 is movably inserted into the bidet assembly 150 but the spray nozzle assembly 160 and the spray canister device 100 are both retracted, and only the air blower wand 165 of the drying nozzle assembly 170 is in an extended position. Figure 7B is a perspective view of the drying nozzle assembly 170, showing the air blower wand 165 in a retracted position within the air blower housing 164. Figure 7C is a partial cross-sectional view of the drying nozzle assembly 170, showing the air blower wand 165 is in an extended position.

Accordingly, in one aspect, the spray nozzle assembly 160 of the bidet assembly 150 includes one or more first steering mechanisms, a first driving motor being connected to one or more spray nozzle wands and adapted for moving the one ore more spray nozzle wands in retracting-and-extending motion. In another aspect, the spray nozzle assembly 160 further includes one or more second steering mechanisms, and a second driving motor being connected to the one or more spray nozzle wands and adapted for moving the one or more spray nozzle wands in three-dimensional circular rotational motion.

For example, the spray nozzle wand 163 of the spray nozzle assembly 160 and the air blower wand 165 of the drying nozzle assembly 170 are adapted to extend and retract along the delivery track 158 between the retracted position and the extended position with the assistance of a steering mechanism 144, motors 133, 135, and motor drive mechanisms 134, 136. Further, one or more motors and one or more motor drive mechanisms 138 are used to drive and provide three-dimensional movements of the spray nozzle wand 163 of the spray nozzle assembly 160 and the air blower wand 165 of the drying nozzle assembly 170.

In one embodiment, the spray nozzle wand 163 of the spray nozzle assembly 160 and the air blower wand 165 of the drying nozzle assembly 170 are adapted to move in three-dimensional movements together. In another embodiment, the spray nozzle wand 163 of the spray nozzle assembly 160 and the air blower wand 165 of the drying nozzle assembly 170 are adapted to move in independent three-dimensional movements.

In one example, the one ore more spray nozzle wands 163 within the spray nozzle housing 162 are retractable and adapted to deliver a second solution to the region of the human body. In another example, the one or more spray nozzle units of the spray nozzle assembly 160 may include a first spray nozzle channel adapted to deliver the second solution and a second spray nozzle channel adapted to deliver a third solution. Examples of the second solution and the third solution include, but are not limited to, water, a cleaning solution, a barrier spray solution, a medicine-containing solution, and combinations thereof, and wherein the third solution is selected form the group consisting of water, a cleaning solution, a barrier spray solution, a medicine-containing solution, and combinations thereof.

One example of the second solution and the third solution is water and a cleanser solution, respectively. Accordingly, the first spray nozzle channel is adapted to deliver water to the region of the human body for washing the region, and wherein the second spray nozzle channel is adapted to deliver a cleaning solution (e.g., a bath foam solution, disinfectant solutions, cleaning solution, *etc.)* for cleaning the region of the human body. Another example of the second solution and the third solution is water for washing the region of the human body and a medicine-containing solution for treating the region cleanser solution, respectively.

In another aspect, the drying nozzle assembly 170 may include a first driving motor being connected to one or more drying nozzle units and adapted for moving one ore more retractable air blower wands in retracting-and-extending motion, and a second driving motor being connected to the one or more drying nozzle unit and adapted for moving the one or more retractable air blower wands in three-dimensional circular rotational motion.

For example, the air blower wand 165 of the drying nozzle assembly 170 is adapted to extend and retract along the delivery track 158 between the retracted position and the extended position with the assistance of the motor 135 and motor drive mechanisms 134. Further, one or more motors and one or more motor drive mechanisms 138 are used to drive the air blower wand 165 and provide three-dimensional movements of the air blower wand 165 of the drying nozzle assembly 170.

In another embodiment, the toilet seat assembly 200 further includes a medicine delivery assembly 190 (as shown in Figure 1 and Figure 2A) with one or more medicine storage cartridges. The medicine delivery assembly 190 is adapted to deliver one or more medicine-containing solutions stored at the one or more medicine storage cartridges. In one aspect, the medicine delivery assembly 190 is coupled to a spray nozzle channel within the spray nozzle wands 163 of the spray nozzle assembly 160. In another aspect, the medicine delivery assembly 190 may additionally include one or more medicine base units, each medicine base unit comprise at least a cartridge unit and at least a cartridges slot, wherein the cartridge slot is adapted to match with a medicine storage cartridge, and wherein the medicine storage cartridge comprises a cartridge cover, a medicine inlet being connected to a medicine storage assembly, and a sensor.

In addition, the apparatus of the invention may further include a power switch, a power indicator for powering up the apparatus and operating wash, dry, clean, protect, and other functions. In one aspect, the apparatus is connected to a power cord. In another aspect, the apparatus is connected to a battery power pack.

Referring back to Figures 1 and 2A-2B, an apparatus is provided for storing a first solution and spraying the first solution onto a region of the human body and includes the toile seat assembly 200 having the seat cover 210 and the bidet seat 230, wherein the bidet seat 230 includes the seat body 232, the seat base 240, the bidet housing 220 for housing the bidet assembly 150 therein, the bidet spray cover 172, and the bidet housing cover 222, wherein the bidet assembly 150 includes a removable spray canister device 100 adapted to store the first solution and be inserted in and out of the base housing cover 222.

In one embodiment, the apparatus further includes the front end touch control unit 344 positioned at a front end of the seat body to control the movements of the bidet assembly, and adapted to control opening and closing movements of the bidet spray cover 172. In one aspect, the bidet spray cover 172 is capable of being in an open position and a closed position and adapted to cover the bidet assembly 150 housed within the bidet housing 220. In another aspect, the bidet housing cover 222 is positioned at a surface of the bidet housing 220.

In another embodiment, the bidet assembly 150 of the apparatus further includes the spray nozzle assembly 160 having a spray nozzle housing 162 adapted to house one or more spray nozzle units comprising one ore more spray nozzle wands 163 with one or more openings on the spray nozzle head 166. In one example, the one ore more spray nozzle wands 163 are retractable and adapted to deliver a second solution to the region of the human body.

In still another embodiment, the bidet assembly 150 of the apparatus further includes the drying nozzle assembly 170 having one or more drying nozzle units and a fan connected to the one or more drying nozzle units. Each drying nozzle unit may include one or more air blower wands 165 and one or more openings on the air blower nozzle head 168. In one example, the one or more air blower wands 165 are retractable and adapted to deliver air at a predetermined temperature to the region of the human body; and

One or more examples of the spray nozzle assembly 160 and/or drying nozzle assembly 170, and the medicine delivery assembly 190 are described in detail in co-pending U.S. patent application serial number 15/588,635, filed May 6, 2017, co-pending U.S. patent application serial number 15/588,637, filed May 6, 2017, co-pending U.S. patent application serial number 15/588,638, filed May 6, 2017, and co-pending U.S. patent application serial number 15/588,640, filed May 6, 2017. The disclosures of these US patent applications are hereby incorporated by reference.

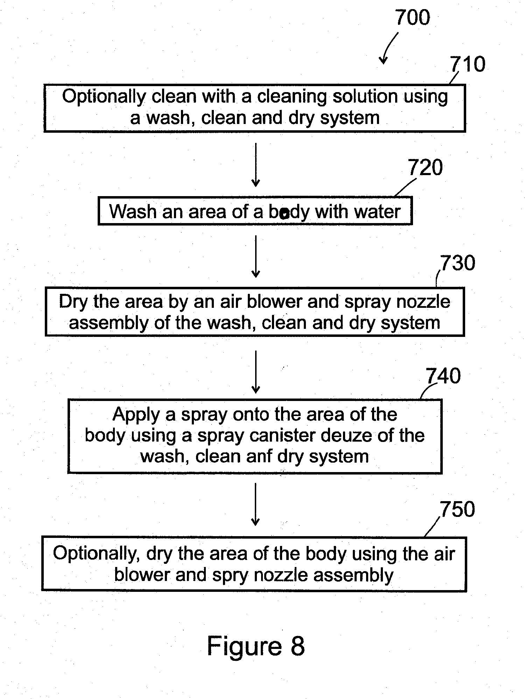

Figure 8 illustrates a method 700 of using a wash, clean and dry system to deliver one or more solutions to a region of a human body. The method 700 may include an optional step of controlling the movements of the bidet assembly and the opening and closing movements of the bidet spray cover using one or more control units.

At Step 710, optionally a surface area of the region of the human body is cleaned with a cleaning solution spraying out from a spray nozzle assembly of a bidet assembly positioned within a bidet housing of a bidet seat of a toilet seat assembly. The cleaning solution may be an enzymatic cleaning solution, a cleanser solution, a bathing foam solution, a soapy solution, or a disinfectant solution. The amount of the cleansing solution is not limited and can be controlled by the human user or automatically set or built-in within one or more control units controlling the wash, clean and dry system. For example, about 5 ml of an enzymatic cleanser solution can be used to cover, pre-wash and clean a desired region of a human body (e.g., genital or anal area, intimate parts, perianal region).

At Step 720, the method 700 includes washing the surface area of the region of the human body with a washing solution (e.g., water or a solution of disinfectant, *etc.),* using the spray nozzle assembly of the toile seat assembly. At this step, the method 700 may include controlling one or more movements of the spray nozzle assembly by one or more control units, directing one or more three-dimensional rotational movements of one or more spray nozzle units of the spray nozzle assembly by communicating one or more control units with one or more first driving motors connected to the one or more spray nozzle units, and directing one or more extending and retracting movements of the one or more spray nozzle units of the spraying nozzle assembly by communicating the one or more control units with one or more second driving motors connected to the one or more spray nozzle units.

The Step 720 may also includes jetting out a cleaning solution from a first nozzle head opening of a first spray nozzle channel within one or more spray nozzle wands of the one or more spray nozzle units, and jetting out a water or saline solution from a second nozzle head opening of a second spray nozzle channel within the one or more spray nozzle wands of the one or more spray nozzle units.

Optionally, during Step 720, a first spray nozzle channel is adapted to deliver water to the region of the human body for washing the region, and a second spray nozzle channel is adapted to deliver a cleaning solution to the region of the human body for cleaning the region. In another aspect, the second spray nozzle channel is adapted to deliver a medicine-containing solution stored within a medicine delivery assembly onto the region of the human body for treating the region.

In still another aspect, a first spray nozzle channel is connected to a first liquid line to deliver a washing solution to the region of the human body for washing the region, and a second spray nozzle channel is connected to a second liquid line to deliver a cleaning solution to the region of the human body for cleaning the region. In yet another aspect, the first spray nozzle channel is connected to a first liquid line to deliver a washing solution to the region of the human body for washing the region, and the second spray nozzle channel is connected to a second liquid line to deliver a medicine-containing solution to the region of the human body for treating the region.

At Step 730, the area of the human body of the user is dried using an drying nozzle assembly of the bidet assembly. The Step 730 may include controlling one or more movements of the drying nozzle assembly by one or more control units, including directing one or more three-dimensional rotational movements of one or more drying nozzle units of the drying nozzle assembly by communicating one or more control units with one or more first driving motors connected to the one or more drying nozzle units, and directing one or more extending and retracting movements of the one or more drying nozzle units of the drying nozzle assembly by communicating the one or more control units with one or more second driving motors connected to the one or more drying nozzle units.

In one embodiment, air from the drying nozzle opening is blown out at a predetermined temperature and a predetermined speed. The Step 730 may also include rotating an air blower nozzle head on the front tip of an air blower wand of the one or more drying nozzle assembly so that the air can be adjusted in a suitable direction to be directed to a desired region of the human body. In addition, one or more retractable elements within the one or more drying nozzle assembly are adjusted to retract and/or extend the air blower wand so that the air is be adjusted to be directed to a desired region of the human body.

In addition, the Step 730 further includes receiving a user input from the drying nozzle assembly from the one or more control units, and adjusting the one or more extending and retracting movements of the one or more drying nozzle unit based on the user input. Further, the Step 730 further includes receiving a user input from the drying nozzle assembly from the one or more control units; and adjusting the one or more three-dimensional rotational movements of the air blower nozzle head and the one or more air blower wands of the drying nozzle assembly based on the user input.

At Step 740, the area of the human body of the user is protected and moisture by spraying a first solution onto the area of the region using the removable spray canister device. The first solution is stored in the removable spray canister device and may contain a skin protection solution or a solution of barrier materials, such as skin protectants, ointments, mineral oil, silicone fluids, dimethicone, cyclomethicone, petrolatum, cod liver oil, lanolin, zinc oxide, talc, calc, cyclomethicone amine, kaolin, topical starch and allantoin, barrier materials, zinc oxide, skin moisturizers, skin lotions, and combinations thereof.

In operation, once the spray canister device is properly installed into the toilet seat assembly 200, either manually by a human user or pre-installed onto the toilet seat assembly of the system, the spray canister device is adapted to be controlled by the control units to execute commands to move and shaken back and froth for several times so that the first solution stored within the canister element can be mixed thoroughly prior to spay a mist or droplets of the first solution out of the spray canister device.

In addition, once the electric power is turned on and the motors within the clean, wash and dry system are commanded by the one or more control units to deliver the first solution of a skin protection material or barrier spray material, *etc.,* stored within the spray canister device via the channels within the spray canister device and spray a mist, a stream or droplets of the first solution out of the openings at the tip of the top cap portion of the sleeved cover element onto a desirable area of a human body. The shape and size of the openings positioned at the tip of the spray canister device are not limited and can be a small opening, a multiple-holed nozzle type element, among others.

Optionally, at Step 750, the area of the region of the human body is dried using the air blower wand of the drying nozzle assembly after applying the spray of the first solution onto the area of the region using the removable spray canister device. Alternatively, the area of the region is dried before applying the spray of the barrier protective solution onto the area of the region using the removable spray canister device. In another embodiment, the area of the region is dried both before and after applying the spray of the first solution onto the area of the region using the removable spray canister device. The condition of drying the area before or after applying the spray of the first solution can be set similarly; however, the speed and temperature of the air blown to the area of the body may differ, and can be adjusted according to personal preference.

In addition, a medicine-containing solution can be applied onto the area using a medicine delivery assembly and/or a spray canister device as described herein. The method may further include providing one or more medicines to a medicine delivery assembly and/or a spray canister device that are coupled to a toilet seat assembly, and controlling one or more movements of the medicine delivery assembly and/or the spray canister device by one or more control units.

In one aspect, controlling the movements of the medicine delivery assembly includes directing one or more extending and retracting movements of a medicine delivery nozzle of the medicine delivery assembly by communicating the one or more control units with one or more first driving motors connected to the medicine delivery nozzle, and directing one or more three-dimensional rotational movements of the medicine delivery nozzle of the medicine delivery assembly by communicating the one or more control units with one or more second motors connected to the medicine delivery nozzle.

The system provided here present a safer, more hygienic, and more effective alternative method to self-administer perianal medicines than any option currently available. To this extent, the system can present a discreet, "hands-free" alternative to the current options, or couple to other system, such as a bidet toilet seat system, thereby substantially eliminating any discomfort, ineffectiveness, and/or embarrassment a user might otherwise experience.

The wash, clean and dry system of the invention can be formed by one or more parts together into a support enclosure in order to contain therein various fluid channels, electric circuits (e.g., printed circuit boards (PCB)), motors, drive mechanism, steering gears, coupling mechanisms, and combinations thereof, among others.

In one embodiment, the wash, dry and clean system described herein may further includes one or more power switches and/or control units such that various motors and electric circuits contained within the toilet seat assembly can be turned on for powering up the system and operating wash, clean, dry, spray and other functions of the system. The power switch can be positioned, in one example, remotely away from the system (but easily visible to an user); on the lap of an user to be easily visible handled by an user; on one side of the toilet seat assembly; or on the front end of the toile seat. In addition, a power indicator can be positioned to indicate turning on of the electric power and proper functioning of the clean, wash and dry system.

In one embodiment, the one or more control units includes a front end touch control unit which is a single button with electric circuits therein to be adapted to control the various movements within the bidet assembly of the toilet seat assembly of the system. In another embodiment, the method of the invention includes repeatedly touching of the single button of the front end touch control unit to direct one or more steps of the method 700 as described herein where the control commands are built-in within the front end touch control unit in a sequential order.

For example, one touch of the single button of the front end touch control unit is directed to Step 710 of cleaning a region of a human body using the bidet assembly of the system herein, whereas another touch (a second touch or two continuous pushes of the single button) of the single button of the front end touch control unit is directed to control the system into Step 720 of washing the region of the human body and directing movements of a spray nozzle assembly within the bidet assembly of the system herein. Likewise, a third touch (two or three continuous pushes, *etc.)* on the single button of the front end touch control unit is directed to execute a command to execute Step 730 of drying the region of the human body and directing the movements of the drying nozzle assembly. Further push of the single button of the front end touch control unit may further control the system to execute Step 740, and/or step 750, depending on system models and built-in functions installed on the system.

In operation, once the electric power is turned on and the motors within the clean, wash and dry system are adapted to deliver all desirable liquids (e.g., water and liquid from a water solution assembly via a water inlet, a cleaning solution inlet and/or a medication liquid solution inlet, and combinations thereof) via one or more channels within the spray nozzle assembly and delivered the desired solutions to the spray nozzle wand. On or more openings on the spray nozzle head are adapted to spray out liquid solutions in a stream or a mist form onto a desirable area of a human body. The shape and size of the openings positioned at the tip of the spray nozzle wand are not limited and can be a small opening, a multiple-holed nozzle type element, among others. Accordingly, a method and apparatus that is easy to carry, easy to be connected to auxiliary parts, and easy to install and use is provided for washing, cleaning and drying a region of a human body.

While the foregoing is directed to embodiments of the present invention, other and further embodiments of the invention may be devised without departing from the basic scope thereof, and the scope thereof is determined by the claims that follow.

## Claims

1. An apparatus for storing a first solution and spraying the first solution onto a region of the human body, comprising:
a toilet seat assembly (200) having a seat cover (210) and a bidet seat (230);
wherein the bidet seat (230) comprises a seat body (232), a seat base (240), a bidet assembly (150) housed within a bidet housing (220) , a bidet spray cover (172), and a bidet housing cover (222);
wherein the bidet housing cover (222) is positioned at a surface of the bidet housing (220);
wherein the bidet spray cover (172) is capable of being in an open position and a closed position and adapted to cover the bidet assembly (150) housed within the bidet housing (220);
wherein the bidet assembly (150) comprises a removable spray canister device (100) adapted to store the first solution and to be inserted in as well as to be taken out of the bidet housing cover (222), and a spray nozzle assembly (160);
wherein the spray nozzle assembly (160) comprises a spray nozzle housing (162) adapted to house one or more spray nozzle units comprising one or more spray nozzle wands (163) that are retractable, and a first driving motor being connected to the one or more spray nozzle wands (163) and adapted for moving the one or more spray nozzle wands (163) in retracting-and-extending motion, **characterized in that** the one or more spray nozzle wands (163) are adapted to deliver a second solution to the region of the human body.

2. The apparatus of claim 1, wherein the removable spray canister device (110) further comprises:
a sleeved cover element (110); and
a canister element (120).

3. The apparatus of claim 2, wherein the sleeved cover element (110) of the removable spray canister device further comprises:
a top cap portion (104); an opening at the top cap portion (104); a sleeve portion (106); and a trigger bar (114), and preferably wherein the first solution is stored within the canister element (120) of the removable spray canister device (100) and the first solution is sprayed out by triggering the trigger bar (114) of the sleeved cover element (110) against the canister element (120) and moving the bottom portion of the canister element (120) to be closer to the opening of the sleeved cover element (120).

4. The apparatus of claim 2, wherein the sleeved cover element (110) further comprises a handle grip (112) adapted to handle the removable spray canister device (100) in and out of the bidet housing cover (222).

5. The apparatus of claim 1 , wherein the first solution is selected form the group consisting of skin protectants, ointments, mineral oil, silicone fluids, dimethicone, cyclomethicone, petrolatum, cod liver oil, lanolin, zinc oxide, talc, calamine, kaolin, topical starch and allantoin, barrier materials, zinc oxide, skin moisturizers, skin lotions, moisturizing creams, skin sealants, water, medication, cleaning solutions, moisture barriers, medicaments, a pharmaceutical formulation, and combinations thereof.

6. The apparatus of claim 1, wherein the spray nozzle assembly (160) further comprises one or more steering mechanisms, and a second driving motor being connected to the one or more spray nozzle wands (163) and adapted for moving the one or more spray nozzle wands (163) in three-dimensional circular rotational motion.

7. The apparatus of claim 1, wherein the one or more spray nozzle units of the spray nozzle assembly (160) further comprise:
a first spray nozzle channel adapted to deliver the second solution; and a second spray nozzle channel adapted to deliver a third solution, wherein the second solution is selected form the group consisting of water, a cleaning solution, a barrier spray solution, a medicine-containing solution, and combinations thereof, and wherein the third solution is selected form the group consisting of water, a cleaning solution, a barrier spray solution, a medicine-containing solution, and combinations thereof.

8. The apparatus of claim 7, wherein the first spray nozzle channel is adapted to deliver water to the region of the human body for washing the region, and wherein the second spray nozzle channel is adapted to deliver a cleaning solution to the region of the human body for cleaning the region, or wherein the second spray nozzle channel is adapted to deliver a medicine containing solution to the region of the human body for treating the region.

9. The apparatus of claim 1, wherein the bidet assembly (150) further comprises a drying nozzle assembly (170) comprising:
one or more drying nozzle units comprising one or more retractable air blower wands (165) adapted to deliver air at a predetermined temperature to the region of the human body; and a fan connected to the one or more drying nozzle units, and preferably wherein the drying nozzle assembly (170) further comprises: a first driving motor being connected to the one or more drying nozzle units and adapted for moving the one or more retractable air blower wands (165) in retracting-and-extending motion; and a second driving motor being connected to the one or more drying nozzle units and adapted for moving the one or more retractable air blower wands in three-dimensional circular rotational motion.

10. The apparatus of claim 1, further comprising a medicine delivery assembly (190) having one or more medicine storage cartridges, wherein the medicine delivery assembly (190) is adapted to deliver one or more medicine-containing solutions stored at the one or more medicine storage cartridges, and preferably wherein the medicine delivery assembly (190) is coupled to a spray nozzle wand (163) of a spray nozzle assembly (160).

11. The apparatus of claim 1, further comprising:
one or more control units (340, 342, and 344) adapted to receive an user input and, based on the user input, to direct movements of the bidet assembly (150) to be retractable out of the bidet housing (220) when the bidet housing cover (222) is in an open position and retractable inside the bidet housing (220) when the bidet housing cover (222) is in a closed position, wherein the one or more control units (340, 342, and 344) is selected from the group consisting of a remote control unit, a touch screen control unit, a joystick type control unit, a handheld control unit, a steering-wheel type control unit, a built-in control unit adjacent the toilet seat assembly, a front end touch control unit, and combinations thereof.

12. The apparatus of claim 11, wherein the front end touch control is positioned at a front end of the seat body (232) to control the movements of the bidet assembly (150), and adapted to control opening and closing movements of the bidet housing cover (220).

13. The apparatus of claim 12, wherein the front end touch control unit is a single button with electric circuits therein to be adapted to control the movements of the bidet assembly (150) and the opening and closing movements of the bidet housing cover (220), wherein the user input comprises repeated touching of the single button.

14. The apparatus of claim 1, wherein the bidet assembly further comprises: a drying nozzle assembly (170) comprising: one or more drying nozzle units comprising one or more retractable air blower wands (165) adapted to deliver air at a predetermined temperature to the region of the human body; and a fan connected to the one or more drying nozzle units.

15. A method of using an apparatus to deliver one or more solutions to a region of a human body, comprising:
washing an area of the region with water using the apparatus, wherein the apparatus comprises:
a toilet seat assembly (200) having a seat cover (210) a bidet seat (230), a seat body (232), a seat base (240), a bidet assembly (150) housed within a bidet housing (220), wherein the bidet assembly (150) comprises a bidet spray cover (172), and a bidet housing cover (222), wherein the bidet assembly (150) comprises: a removable spray canister device (100), a spray nozzle assembly (160), and a drying nozzle assembly (170);
drying the area of the region using a drying nozzle assembly (170) of the bidet assembly (150);
and applying a spray of a first solution onto the area of the region using the removable spray canister device (100) which stores the first solution therein.

16. The method of claim 15, wherein the spray of the first solution stored in the removable spray canister device is selected form the group consisting of skin protectants, ointments, mineral oil, silicone fluids, dimethicone, cyclomethicone, petrolatum, cod liver oil, lanolin, zinc oxide, talc, calamine, kaolin, topical starch and allantoin, barrier materials, zinc oxide, skin moisturizers, skin lotions, moisturizing creams, skin sealants, water, medication, cleaning solutions, moisture barriers, medicaments, a pharmaceutical formulation, and combinations thereof.

17. The method of claim 15, further comprising: drying the area of the region after applying the spray of the first solution onto the area of the region using the removable spray canister device (100).

18. The method of claim 15, further comprising:
controlling the movements of the bidet assembly (150) and the opening and closing movements of the bidet housing cover (222) using one or more control units (340, 342, and 344), and preferably wherein the one or more control units (340, 342, and 344) comprises a front end touch control unit which is a single button with electric circuits therein to be adapted to control, and most preferably the method further comprising:
repeatedly touching of the single button of the front end touch control.

## Patentansprüche

1. Einrichtung zum Lagern einer ersten Lösung und zum Sprühen der ersten Lösung auf einen Bereich des menschlichen Körpers, umfassend:
eine Toilettensitzbaugruppe (200) mit einer Sitzabdeckung (210) und einem Bidetsitz (230);
wobei der Bidetsitz (230) einen Sitzkörper (232), eine Sitzbasis (240), eine in einem Bidetgehäuse (220) aufgenommene Bidetbaugruppe (150), eine Bidetsprühabdeckung (172) und eine Bidetgehäuseabdeckung (222) umfasst;
wobei die Bidetgehäuseabdeckung (222) an einer Oberfläche des Bidetgehäuses (220) positioniert ist;
wobei die Bidetsprühabdeckung (172) in einer offenen Position und einer geschlossenen Position sein kann und ausgelegt ist, um die in dem Bidetgehäuse (220) aufgenommene Bidetbaugruppe (150) abzudecken;
wobei die Bidetbaugruppe (150) eine abnehmbare Sprühkanistervorrichtung (100) umfasst, die ausgelegt ist, um die erste Lösung zu lagern und in die Bidetgehäuseabdeckung (222) eingesetzt sowie aus dieser herausgenommen zu werden, und eine Sprühdüsenbaugruppe (160);
wobei die Sprühdüsenbaugruppe (160) ein Sprühdüsengehäuse (162) umfasst, das ausgelegt ist, um eine oder mehrere Sprühdüseneinheiten aufzunehmen, die einen oder mehrere Sprühdüsenstäbe (163) umfassen, die einziehbar sind,
und einen ersten Antriebsmotor, der mit dem einen oder den mehreren Sprühdüsenstäben (163) verbunden und ausgelegt ist, um den einen oder die mehreren Sprühdüsenstäbe (163) in einer Einzieh- und Ausfahrbewegung zu bewegen, **dadurch gekennzeichnet, dass** der eine oder die mehreren Sprühdüsenstäbe (163) ausgelegt sind, um eine zweite Lösung an den Bereich des menschlichen Körpers abzugeben.

2. Einrichtung nach Anspruch 1, wobei die abnehmbare Sprühkanistervorrichtung (110) ferner Folgendes umfasst:
ein ummanteltes Abdeckelement (110); und
ein Kanisterelement (120).

3. Einrichtung nach Anspruch 2, wobei das ummantelte Abdeckelement (110) der abnehmbaren Sprühkanistervorrichtung ferner Folgendes umfasst:
einen oberen Kappenabschnitt (104); eine Öffnung am oberen Kappenabschnitt (104); einen Hülsenabschnitt (106); und eine Auslösestange (114), und wobei vorzugsweise die erste Lösung innerhalb des Kanisterelements (120) der abnehmbaren Sprühkanistervorrichtung (100) gelagert ist und die erste Lösung durch Auslösen der Auslösestange (114) des ummantelten Abdeckelements (110) gegen das Kanisterelement (120) und Bewegen des unteren Abschnitts des Kanisterelements (120), um näher an der Öffnung des ummantelten Abdeckelements (120) zu sein, herausgesprüht wird.

4. Einrichtung nach Anspruch 2, wobei das ummantelte Abdeckelement (110) ferner einen Handgriff (112) umfasst, der ausgelegt ist, um die abnehmbare Sprühkanistervorrichtung (100) innerhalb und außerhalb der Bidetgehäuseabdeckung (222) zu handhaben.

5. Einrichtung nach Anspruch 1, wobei die erste Lösung aus der Gruppe ausgewählt ist, die aus Hautschutzmitteln, Salben, Mineralöl, Silikonfluiden, Dimethicon, Cyclomethicon, Petrolatum, Lebertran, Lanolin, Zinkoxid, Talkum, Galmei, Kaolin, topischer Stärke und Allantoin, Barrierematerialien, Zinkoxid, Hautfeuchtigkeitsmitteln, Hautlotionen, Feuchtigkeitscremes, Hautversiegelungsmitteln, Wasser, Medikation, Reinigungslösungen, Feuchtigkeitsbarrieren, Medikamenten, einer pharmazeutischen Formulierung und Kombinationen davon besteht.

6. Einrichtung nach Anspruch 1, wobei die Sprühdüsenbaugruppe (160) ferner einen oder mehrere Lenkmechanismen und einen zweiten Antriebsmotor umfasst, der mit dem einen oder den mehreren Sprühdüsenstäben (163) verbunden und zum Bewegen des einen oder der mehreren Sprühdüsenstäbe (163) in einer dreidimensionalen kreisförmigen Rotationsbewegung ausgelegt ist.

7. Einrichtung nach Anspruch 1, wobei die eine oder mehreren Sprühdüseneinheiten der Sprühdüsenbaugruppe (160) ferner Folgendes umfassen:
einen ersten Sprühdüsenkanal, der ausgelegt ist, um die zweite Lösung abzugeben; und einen zweiten Sprühdüsenkanal, der ausgelegt ist, um eine dritte Lösung abzugeben, wobei die zweite Lösung aus der Gruppe ausgewählt ist, die aus Wasser, einer Reinigungslösung, einer Barrieresprühlösung, einer medikamentenhaltigen Lösung und Kombinationen davon besteht, und wobei die dritte Lösung aus der Gruppe ausgewählt ist, die aus Wasser, einer Reinigungslösung, einer Barrieresprühlösung, einer medikamentenhaltigen Lösung und Kombinationen davon besteht.

8. Einrichtung nach Anspruch 7, wobei der erste Sprühdüsenkanal ausgelegt ist, um Wasser an den Bereich des menschlichen Körpers zum Waschen des Bereichs abzugeben, und wobei der zweite Sprühdüsenkanal ausgelegt ist, um eine Reinigungslösung an den Bereich des menschlichen Körpers zum Reinigen des Bereichs abzugeben, oder wobei der zweite Sprühdüsenkanal ausgelegt ist, um eine medikamentenhaltige Lösung an den Bereich des menschlichen Körpers zum Behandeln des Bereichs abzugeben.

9. Einrichtung nach Anspruch 1, wobei die Bidetbaugruppe (150) ferner eine Trocknungsdüsenbaugruppe (170) umfasst, die Folgendes umfasst:
eine oder mehrere Trocknungsdüseneinheiten, die einen oder mehrere einziehbare Luftgebläsestäbe (165) umfassen, die ausgelegt sind, um Luft mit einer vorbestimmten Temperatur an den Bereich des menschlichen Körpers abzugeben; und ein Gebläse, das mit der einen oder den mehreren Trocknungsdüseneinheiten verbunden ist, und wobei die Trocknungsdüsenbaugruppe (170) vorzugsweise ferner Folgendes umfasst: einen ersten Antriebsmotor, der mit der einen oder den mehreren Trocknungsdüseneinheiten verbunden und zum Bewegen der einen oder mehreren einziehbaren Luftgebläsestäbe (165) in einer Einzieh- und Ausfahrbewegung ausgelegt ist; und einen zweiten Antriebsmotor, der mit der einen oder den mehreren Trocknungsdüseneinheiten verbunden und zum Bewegen der einen oder mehreren einziehbaren Luftgebläsestäbe in einer dreidimensionalen kreisförmigen Rotationsbewegung ausgelegt ist.

10. Einrichtung nach Anspruch 1, ferner umfassend eine Medikamentenabgabebaugruppe (190) mit einer oder mehreren Medikamentenlagerkartuschen, wobei die Medikamentenabgabebaugruppe (190) ausgelegt ist, um eine oder mehrere medikamentenhaltige Lösungen abzugeben, die in der einen oder den mehreren Medikamentenlagerkartuschen gelagert sind, und wobei vorzugsweise die Medikamentenabgabebaugruppe (190) an einen Sprühdüsenstab (163) einer Sprühdüsenbaugruppe (160) gekoppelt ist.

11. Einrichtung nach Anspruch 1, ferner umfassend:
eine oder mehrere Steuereinheiten (340, 342 und 344), die ausgelegt sind, um eine Benutzereingabe zu empfangen und auf Grundlage der Benutzereingabe Bewegungen der Bidetbaugruppe (150) zu lenken, damit sie aus dem Bidetgehäuse (220) einziehbar ist, wenn die Bidetgehäuseabdeckung (222) in einer offenen Position ist, und in das Bidetgehäuse (220) einziehbar ist, wenn sich die Bidetgehäuseabdeckung (222) in einer geschlossenen Position befindet, wobei die eine oder mehreren Steuereinheiten (340, 342 und 344) aus der Gruppe ausgewählt sind, die aus einer Fernbedienungseinheit, einer Berührungsbildschirm-Steuereinheit, einer Joystick-Steuereinheit, einer in der Hand gehaltenen Steuereinheit, einer Steuerrad-Steuereinheit, einer eingebauten Steuereinheit neben der Toilettensitzbaugruppe, einer Steuereinheit mit Vorderseitenberührung und Kombinationen davon besteht.

12. Einrichtung nach Anspruch 11, wobei die Steuerung mit Vorderseitenberührung an einem vorderen Ende des Sitzkörpers (232) positioniert ist, um die Bewegungen der Bidetbaugruppe (150) zu steuern, und ausgelegt ist, um Öffnungs- und Schließbewegungen der Bidetgehäuseabdeckung (220) zu steuern.

13. Einrichtung nach Anspruch 12, wobei die Steuereinheit mit Vorderseitenberührung eine einzelne Taste mit darin befindlichen elektrischen Schaltungen ist, die ausgelegt sind, um die Bewegungen der Bidetbaugruppe (150) und die Öffnungs- und Schließbewegungen der Bidetgehäuseabdeckung (220) zu steuern, wobei die Benutzereingabe ein wiederholtes Berühren der einzelnen Taste umfasst.

14. Einrichtung nach Anspruch 1, wobei die Bidetbaugruppe ferner Folgendes umfasst: eine Trocknungsdüsenbaugruppe (170), umfassend: eine oder mehrere Trocknungsdüseneinheiten, die eine oder mehrere einziehbare Luftgebläsestäbe (165) umfassen, die ausgelegt sind, um Luft mit einer vorbestimmten Temperatur an den Bereich des menschlichen Körpers abzugeben; und ein Gebläse, das mit der einen oder den mehreren Trocknungsdüseneinheiten verbunden ist.

15. Verfahren zum Verwenden einer Einrichtung zum Abgeben einer oder mehrerer Lösungen an einen Bereich eines menschlichen Körpers, umfassend:
Waschen einer Fläche des Bereichs mit Wasser unter Verwendung der Einrichtung, wobei die Einrichtung Folgendes umfasst:
eine Toilettensitzbaugruppe (200) mit einer Sitzabdeckung (210), einem Bidetsitz (230), einem Sitzkörper (232), einer Sitzbasis (240), einer Bidetbaugruppe (150), die in einem Bidetgehäuse (220) aufgenommen ist, wobei die Bidetbaugruppe (150) eine Bidetsprühabdeckung (172) umfasst, und einer Bidetgehäuseabdeckung (222), wobei die Bidetbaugruppe (150) Folgendes umfasst: eine abnehmbare Sprühkanistervorrichtung (100), eine Sprühdüsenbaugruppe (160) und eine Trocknungsdüsenbaugruppe (170);
Trocknen der Fläche des Bereichs unter Verwendung einer Trocknungsdüsenbaugruppe (170) der Bidetbaugruppe (150);
und Auftragen eines Sprays einer ersten Lösung auf die Fläche des Bereichs unter Verwendung der abnehmbaren Sprühkanistervorrichtung (100), die die erste Lösung darin lagert.

16. Verfahren nach Anspruch 15, wobei das Spray der ersten Lösung, die in der abnehmbaren Sprühkanistervorrichtung gelagert ist, aus der Gruppe ausgewählt ist, die aus Hautschutzmitteln, Salben, Mineralöl, Silikonfluiden, Dimethicon, Cyclomethicon, Petrolatum, Lebertran, Lanolin, Zinkoxid, Talkum, Galmei, Kaolin, topischer Stärke und Allantoin, Barrierematerialien, Zinkoxid, Hautfeuchtigkeitsmitteln Hautlotionen, Feuchtigkeitscremes, Hautversiegelungsmitteln, Wasser, Medikation, Reinigungslösungen, Feuchtigkeitsbarrieren, Medikamenten, einer pharmazeutischen Formulierung und Kombinationen davon besteht.

17. Verfahren nach Anspruch 15, ferner umfassend: Trocknen der Fläche des Bereichs nach dem Auftragen des Sprays der ersten Lösung auf die Fläche des Bereichs unter Verwendung der abnehmbaren Sprühkanistervorrichtung (100).

18. Verfahren nach Anspruch 15, ferner umfassend:
Steuern der Bewegungen der Bidetbaugruppe (150) und der Öffnungs- und Schließbewegungen der Bidetgehäuseabdeckung (222) unter Verwendung einer oder mehrerer Steuereinheiten (340, 342 und 344), und wobei vorzugsweise die eine oder mehreren Steuereinheiten (340, 342 und 344) eine Steuereinheit mit Vorderseitenberührung umfassen, die eine einzelne Taste mit elektrischen Schaltungen darin ist, damit sie zum Steuern ausgelegt ist, und das Verfahren am meisten bevorzugt ferner Folgendes umfasst:
wiederholtes Berühren der einzelnen Taste der Steuerung mit Vorderseitenberührung.

## Revendications

1. Appareil pour stocker une première solution et pulvériser la première solution sur une région du corps humain, comprenant :
un ensemble siège de toilette (200) ayant un couvercle de siège (210) et un siège de bidet (230) ;
dans lequel le siège de bidet (230) comprend un corps de siège (232), une base de siège (240), un ensemble bidet (150) logé à l'intérieur d'un boîtier de bidet (220), un couvercle de pulvérisation de bidet (172) et un couvercle de boîtier de bidet (222) ;
dans lequel le couvercle de boîtier de bidet (222) est positionné au niveau d'une surface du boîtier de bidet (220) ;
dans lequel le couvercle de pulvérisation de bidet (172) est capable d'être dans une position ouverte et une position fermée et conçu pour couvrir l'ensemble bidet (150) logé à l'intérieur du boîtier de bidet (220) ;
dans lequel l'ensemble bidet (150) comprend un dispositif de bombe de pulvérisation amovible (100) conçu pour stocker la première solution et pour être inséré ainsi que pour être retiré du couvercle de boîtier de bidet (222),
et un ensemble buse de pulvérisation (160) ;
dans lequel l'ensemble buse de pulvérisation (160) comprend un boîtier de buse de pulvérisation (162) conçu pour loger une ou plusieurs unités de buse de pulvérisation comprenant une ou plusieurs tiges de buse de pulvérisation (163) qui sont rétractables,
et un premier moteur d'entraînement étant connecté aux une ou plusieurs tiges de buse de pulvérisation (163) et conçu pour déplacer les une ou plusieurs tiges de buse de pulvérisation (163) dans un mouvement de rétraction et d'extension, **caractérisé en ce que** les une ou plusieurs tiges de buse de pulvérisation (163) sont conçues pour fournir une deuxième solution à la région du corps humain.

2. Appareil selon la revendication 1, dans lequel le dispositif de bombe de pulvérisation amovible (110) comprend en outre :
un élément de couvercle à manchon (110) ; et
un élément de bombe (120).

3. Appareil selon la revendication 2, dans lequel l'élément de couvercle à manchon (110) du dispositif de bombe de pulvérisation amovible comprend en outre :
une partie de capuchon supérieur (104) ; une ouverture au niveau de la partie de capuchon supérieur (104) ; une partie de manchon (106) ; et une barre de déclenchement (114), et de préférence dans lequel la première solution est stockée à l'intérieur de l'élément de bombe (120) du dispositif de bombe de pulvérisation amovible (100) et la première solution est pulvérisée en déclenchant la barre de déclenchement (114) de l'élément de couvercle à manchon (110) contre l'élément de bombe (120) et en déplaçant la partie inférieure de l'élément de bombe (120) pour qu'elle soit plus proche de l'ouverture de l'élément de couvercle à manchon (120).

4. Appareil selon la revendication 2, dans lequel l'élément de couvercle à manchon (110) comprend en outre une poignée (112) conçue pour manipuler le dispositif de bombe de pulvérisation amovible (100) dans et hors du couvercle de boîtier de bidet (222).

5. Appareil selon la revendication 1, dans lequel la première solution est choisie dans le groupe constitué des protecteurs cutanés, pommades, huile minérale, fluides de silicone, diméthicone, cyclométhicone, vaseline, huile de foie de morue, lanoline, oxyde de zinc, talc, calamine, kaolin, amidon topique et allantoïne, matériaux de barrière, oxyde de zinc, hydratants pour la peau, lotions pour la peau, crèmes hydratantes, scellants pour la peau, eau, médicaments, solutions de nettoyage, barrières contre l'humidité, médicaments, formulation pharmaceutique et combinaisons de ceux-ci.

6. Appareil selon la revendication 1, dans lequel l'ensemble buse de pulvérisation (160) comprend en outre un ou plusieurs mécanismes de direction, et un second moteur d'entraînement étant connecté aux une ou plusieurs tiges de buse de pulvérisation (163) et conçu pour déplacer les une ou plusieurs tiges de buse de pulvérisation (163) dans un mouvement de rotation circulaire tridimensionnel.

7. Appareil selon la revendication 1, dans lequel les une ou plusieurs unités de buse de pulvérisation de l'ensemble buse de pulvérisation (160) comprennent en outre :
un premier canal de buse de pulvérisation conçu pour fournir la deuxième solution ; et un second canal de buse de pulvérisation conçu pour fournir une troisième solution, dans lequel la deuxième solution est choisie dans le groupe constitué de l'eau, d'une solution de nettoyage, d'une solution de pulvérisation barrière, d'une solution contenant un médicament et de combinaisons de celles-ci, et dans lequel la troisième solution est choisie dans le groupe constitué de l'eau, d'une solution de nettoyage, d'une solution de pulvérisation barrière, d'une solution contenant un médicament et de combinaisons de celles-ci.

8. Appareil selon la revendication 7, dans lequel le premier canal de buse de pulvérisation est conçu pour fournir de l'eau à la région du corps humain pour laver la région, et dans lequel le second canal de buse de pulvérisation est conçu pour fournir une solution de nettoyage à la région du corps humain pour nettoyer la région, ou dans lequel le second canal de buse de pulvérisation est conçu pour fournir une solution contenant un médicament à la région du corps humain pour traiter la région.

9. Appareil selon la revendication 1, dans lequel l'ensemble bidet (150) comprend en outre un ensemble buse de séchage (170) comprenant :
une ou plusieurs unités de buse de séchage comprenant une ou plusieurs tiges de soufflage d'air rétractables (165) conçues pour fournir de l'air à une température prédéterminée à la région du corps humain ; et une soufflante connectée aux une ou plusieurs unités de buse de séchage, et de préférence dans lequel l'ensemble buse de séchage (170) comprend en outre : un premier moteur d'entraînement étant connecté aux une ou plusieurs unités de buse de séchage et conçu pour déplacer les une ou plusieurs tiges de soufflage d'air rétractables (165) dans un mouvement de rétraction et d'extension ; et un second moteur d'entraînement étant connecté aux une ou plusieurs unités de buse de séchage et conçu pour déplacer les une ou plusieurs tiges de soufflage d'air rétractables dans un mouvement de rotation circulaire tridimensionnel.

10. Appareil selon la revendication 1, comprenant en outre un ensemble de distribution de médicament (190) ayant une ou plusieurs cartouches de stockage de médicament, dans lequel l'ensemble de distribution de médicament (190) est conçu pour distribuer une ou plusieurs solutions contenant un médicament stockées au niveau des une ou plusieurs cartouches de stockage de médicament, et de préférence dans lequel l'ensemble de distribution de médicament (190) est couplé à une tige de buse de pulvérisation (163) d'un ensemble buse de pulvérisation (160).

11. Appareil selon la revendication 1, comprenant en outre :
une ou plusieurs unités de commande (340, 342 et 344) conçues pour recevoir une entrée utilisateur et, sur la base de l'entrée utilisateur, pour diriger les mouvements de l'ensemble bidet (150) pour qu'il soit rétractable hors du boîtier de bidet (220) lorsque le couvercle de boîtier de bidet (222) est dans une position ouverte et peut être rétracté à l'intérieur du boîtier de bidet (220) lorsque le couvercle de boîtier de bidet (222) est dans une position fermée, dans lequel les une ou plusieurs unités de commande (340, 342 et 344) sont choisies dans le groupe constitué d'une télécommande, d'une unité de commande à écran tactile, d'une unité de commande de type joystick, d'une unité de commande portative, d'une unité de commande de type volant, d'une unité de commande intégrée adjacente à l'ensemble siège de toilette, d'une unité de commande tactile frontale et des combinaisons de celles-ci.

12. Appareil selon la revendication 11, dans lequel la commande tactile frontale est positionnée à une extrémité avant du corps de siège (232) pour commander les mouvements de l'ensemble bidet (150), et conçue pour commander les mouvements d'ouverture et de fermeture du couvercle de boîtier de bidet (220).

13. Appareil selon la revendication 12, dans lequel l'unité de commande tactile frontale est un bouton unique avec des circuits électriques à l'intérieur pour être conçu pour commander les mouvements de l'ensemble bidet (150) et les mouvements d'ouverture et de fermeture du couvercle de boîtier de bidet (220), dans lequel l'entrée utilisateur comprend un toucher répété du bouton unique.

14. Appareil selon la revendication 1, dans lequel l'ensemble bidet comprend en outre : un ensemble buse de séchage (170) comprenant : une ou plusieurs unités de buse de séchage comprenant une ou plusieurs tiges de soufflage d'air rétractables (165) conçues pour fournir de l'air à une température prédéterminée à la région du corps humain ; et une soufflante connectée aux une ou plusieurs unités de buse de séchage.

15. Procédé d'utilisation d'un appareil pour fournir une ou plusieurs solutions à une région d'un corps humain, comprenant :
le lavage d'une zone de la région avec de l'eau à l'aide de l'appareil, dans lequel l'appareil comprend :
un ensemble siège de toilette (200) ayant un couvercle de siège (210), un siège de bidet (230), un corps de siège (232), une base de siège (240), un ensemble bidet (150) logé à l'intérieur d'un boîtier de bidet (220), dans lequel l'ensemble bidet (150) comprend un couvercle de pulvérisation de bidet (172) et un couvercle de boîtier de bidet (222), dans lequel l'ensemble bidet (150) comprend : un dispositif de bombe de pulvérisation amovible (100), un ensemble buse de pulvérisation (160), et un ensemble buse de séchage (170) ;
le séchage de la zone de la région à l'aide d'un ensemble buse de séchage (170) de l'ensemble bidet (150) ; et
l'application d'une pulvérisation d'une première solution sur la zone de la région à l'aide du dispositif de bombe de pulvérisation amovible (100) qui y stocke la première solution.

16. Procédé selon la revendication 15, dans lequel la pulvérisation de la première solution stockée dans le dispositif de bombe de pulvérisation amovible est choisie dans le groupe constitué des protecteurs cutanés, pommades, huile minérale, fluides de silicone, diméthicone, cyclométhicone, vaseline, huile de foie de morue, lanoline, oxyde de zinc, talc, calamine, kaolin, amidon topique et allantoïne, matériaux de barrière, oxyde de zinc, hydratants pour la peau, lotions pour la peau, crèmes hydratantes, scellants pour la peau, eau, médicaments, solutions de nettoyage, barrières contre l'humidité, médicaments, formulation pharmaceutique et des combinaisons de ceux-ci.

17. Procédé selon la revendication 15, comprenant en outre :
le séchage de la zone de la région après l'application de la pulvérisation de la première solution sur la zone de la région à l'aide du dispositif de bombe de pulvérisation amovible (100).

18. Procédé selon la revendication 15, comprenant en outre :
la commande des mouvements de l'ensemble bidet (150) et des mouvements d'ouverture et de fermeture du couvercle de boîtier de bidet (222) à l'aide d'une ou de plusieurs unités de commande (340, 342 et 344), et de préférence dans lequel les une ou plusieurs unités de commande (340, 342 et 344) comprennent une unité de commande tactile frontale qui est un bouton unique avec des circuits électriques à l'intérieur pour être conçu pour commander, et le plus préférablement le procédé comprenant en outre :
le toucher répété du bouton unique de la commande tactile frontale.
